# EUROPEAN PATENT APPLICATION

(11) **EP 3 792 932 A1**
(43) Date of publication of application: **17.03.2021**
(21) Application number: 20194404.8
(22) Date of filing: 03.09.2020
(51) Int. Cl.: G16H 50/20, G16H 40/67, A61B 5/00

(54) **PERSONALIZED VITAL SIGN MONITORS**

(30) Priority: 13.09.2019 US 201962900179 P
(71) Applicant: Hill-Rom Services, Inc., Batesville, IN 47006-9167 (US)
(72) Inventor: KAYSER, Susan A., Batesville, IN 47006-9167 (US); DE BIE, Johannes, Batesville, IN 47006-9167 (US); FITZGIBBONS, Stacey Ann, Batesville, IN 47006-9167 (US); SEPEHR, Reyhaneh, Batesville, IN 47006-9167 (US); NOFFKE, Patrick J., Batesville, IN 47006-9167 (US); MEYERSON, Craig M., Batesville, IN 47006-9167 (US)
(74) Representative: Findlay, Alice Rosemary

(57) **Abstract**

Systems, methods, and devices relate to monitoring biological conditions (e.g., vital signs) using patient-specific ranges. An example method includes receiving, from a sensor, first measurements of a vital sign of an individual determined automatically during a first time period and determining a baseline of the vital sign based on the first measurements by averaging the first measurements. A threshold of the vital sign can be a predetermined percentage of the baseline. Second measurements of the vital sign can be received from the sensor. In response to determining that at least one of the second measurements is outside of the range, an alarm may be triggered.

## Description

### TECHNICAL FIELD

This disclosure relates to vital sign monitors and, in particular, vital sign monitors configured to provide alarms or other indications when patient vital signs fall outside of a personalized range.

In inpatient clinical environments outside of an Intensive Care Unit (ICU), a single care provider may be responsible for monitoring multiple patients. In some cases, the care provider may actively measure vital signs of the patients and compare the vital signs to predetermined ranges that are consistent with healthy individuals. When a patient's vital signs are outside of a personalized range for that patient, the care provider may initiate additional medical treatment for the patient. For instance, the vital signs may indicate that the patient requires critical care, and the care provider may transfer the patient to an ICU. In the ICU, the patient may be monitored continuously by a devoted care provider.

However, as the number of patients that the care provider is responsible for increases, the care provider may measure vital signs less frequently. In non-emergency conditions, a care provider may measure a patient's vital signs once every eight hours, in some cases. Although measuring the patient's vital signs at this frequency can enable the care provider to monitor other patients as well, a patient's condition may deteriorate rapidly between the eight-hour sampling period. As a result, the care provider may be less likely to catch early signs of patient deterioration, and the care provider may be unable to initiate early interventions for a deteriorating patient.

Automated sensors configured to automatically determine patient vital signs (without user intervention) can assist care providers with large patient caseloads. Unlike manual vital sign measurements, automated sensors can measure a patient's vital signs without the presence or intervention of the care provider. However, the fidelity of vital sign determinations made by automated sensors can be significantly lower than the quality of vital sign measurements taken directly by care providers. For instance, in cases of measuring heart rate measurements, automated sensors may measure a patient's heart rate regardless of the patient's physical position or sensor placement. In contrast, a care provider may ensure that a patient is in a relaxed physical position and that the placement of sensors is ideal before measuring the patient's heart rate. Accordingly, some measurements measured by automated sensors are more likely to be inaccurate than measurements measured directly by care providers.

In some cases, automated sensors can measure vital signs semi-continuously, such as once every minute. However, many of these measurements may be inaccurate. When the care provider is notified of every measurement that deviates from a particular threshold, the noise in the measurements may increase the risk that a care provider may be called to a patient's bedside when the patient is not in distress. The noise may also increase the risk that a care provider is not notified when the patient is in distress. These risks are further exacerbated by natural vital sign variations between healthy patients and the relatively low-fidelity measurement performance of automated sensors.

Various implementations of the present disclosure relate to vital sign monitors triggered in response to vital signs falling outside of patient-specific ranges. A baseline vital sign can be identified for an individual, and a personalized range associated with that baseline vital sign can be further identified. A system continuously (or semi-continuously) monitoring the individual's vital sign can alert a care provider when the individual's vital sign is outside of the personalized range. Accordingly, if the individual's baseline is unusual (e.g., greater than or less than a population average), the vital sign monitor may not trigger a false alarm when the individual's vital sign is relatively close to the individual's baseline, even if the vital sign is outside of a healthy range defined for the population. Furthermore, the vital sign monitor may trigger an alarm when the individual's vital sign is relatively far from the individual's baseline, even if the vital sign is in a health range as defined for the population. Thus, both the sensitivity and specificity of vital sign monitors can be enhanced, according to various implementations.

The baseline vital sign can be identified in any number of different ways. In some cases, the baseline vital sign is identified by averaging various vital sign measurements of the individual during a time period in which the individual's vital sign is relatively stable. In some instances, the baseline vital sign is a median of the vital sign measurements. In some cases, the vital sign is considered stable when a variance of vital sign measurements during the time period is less than a threshold variance. The threshold variance can be, for example, a fixed amount or a percentage of the individual's average vital sign during the time period. As used herein, the term "average" may refer to an arithmetic mean. In some instances, the baseline vital sign is determined based on previous measurements of the vital sign during previous hospitalizations, intake procedures, primary care visits, or the like. According to various implementations, the baseline vital sign may be determined based on one or more measurements of the individual's vital sign when the individual is medically stable.

In some cases, the baseline vital sign can be selectively identified when other features indicate that the individual's vital sign is likely to be stable. For example, the baseline vital sign can be identified based on measurements taken when the individual is in a fixed position (e.g., a supine position). In some cases, the baseline vital sign can be identified based on measurements taken when the individual is moving less than a threshold amount. The individual's position and/or movement can be identified using at least one of an accelerometer attached to the individual's body, a gyroscope attached to the individual's body, a pressure sensor in a bed of the individual, or the like. In various implementations, the individual may be administered a medication or other treatment that can render the individual's vital sign measurements unstable. In some examples, the baseline may be identified based on measurements taken more than a particular amount of time (e.g., one or more hours dependent on the pharmacokinetics of the medication, dosage, and dosage form and/or a measured bioavailability of the medication) after the medication or other treatment was administered.

The personalized range can be defined according to an upper threshold and a lower threshold. In some cases, at least one of the upper threshold or the lower threshold can be identified according to the baseline vital sign. For instance, the lower threshold can be a fixed amount less than the baseline vital sign, a predetermined percentage (e.g., less than 100%) of the baseline vital sign, a predetermined percentage (e.g., 50%) between the baseline and a minimum of the vital sign, or a combination thereof. In some examples, the upper threshold can be a fixed amount greater than the baseline vital sign, a predetermined percentage (e.g., greater than 100%) of the baseline vital sign, a predetermined percentage (e.g., 50%) between the baseline and a maximum of the baseline vital sign, or a combination thereof. In various implementations, both the lower threshold and the upper threshold can be identified based on the baseline vital sign. In some cases, one of the lower threshold and the upper threshold is identified based on population values, rather than the individual's baseline. For example, the lower threshold and/or the upper threshold can be the same for all individuals in a population.

In various implementations, a sensor can measure the individual's vital sign. A monitoring system can compare the measured vital sign to the personalized range for the individual. If the system determines that the individual's vital sign falls outside of the personalized range, the system can trigger an alarm. In some cases, the system may only trigger the alarm if the individual's vital sign falls outside of the personalized range for a predetermined amount of time, or for a predetermined number of measurements, which can prevent false alarms.

In various examples, the system may also monitor a rate-of-change of the vital sign measurements and trigger the alarm based on the rate-of-change. The rate-of-change can be an average rate of change based on multiple vital sign measurements, in some cases. In some implementations, the system can compare the rate-of-change to a range and may trigger the alarm when the rate-of-change falls outside of the range. In various examples, the system may selectively trigger the alarm when the system identifies that the measured vital sign of the individual may be outside of a vital sign range and the rate-of-change may be outside of a rate-of-change range. The range may be set in advance by a care provider, set automatically based on historical trends of the individual being monitored, set automatically based on previous analyses (e.g., based on previous studies indicating rates-of-change that are likely to correlate to dangerous conditions, like heart attacks), or the like. In some cases, the system may selectively trigger the alarm when the measured vital sign may be below the vital sign range and the rate-of-change may be below a first threshold, or when the measured vital sign may be above the vital sign range and the rate of change may be above a second threshold. Accordingly, the system can trigger the alarm based on observed trends of the measured vital sign over time.

When the alarm is triggered, an electronic device may output a warning and/or an alert to at least one care provider (e.g., a nurse, physician, or the like). In some cases, the warning and/or the alert can indicate a location and/or identity of the individual.

Various vital signs can be monitored in accordance with implementations of the present disclosure. For instance, at least one of a heart rate, a blood pressure, a blood oxygen saturation percentage, a respiration rate, a core temperature, or a peripheral temperature can be monitored.

The invention will now be further described by way of example with reference to the accompanying drawings, in which:
FIG. 1 illustrates an example environment for continuously monitoring vital signs of multiple patients.
FIG. 2 illustrates an example of a visual interface output by a clinical device.
FIGS. 3A and 3B illustrate examples of vital sign measurements taken semi-continuously over periods of time.
FIGS. 4A and 4B illustrate examples of vital sign measurements and rates-of-change of an individual.
FIG. 5 illustrates an example process for identifying a personalized range of biological condition measurements that can be used to monitor an individual.
FIG. 6 illustrates an example process for triggering an alarm based on measurements of a biological condition.
FIG. 7 illustrates an example system including at least one device. In some implementations, the system illustrated in FIG. 7 may perform any of the functionality described herein.

Various implementations of the present disclosure will be described in detail with reference to the accompanying figures, wherein like reference numerals present like parts and assemblies throughout the several views. Additionally, any samples set forth in this specification are not intended to be limiting and merely set forth some of the many possible implementations.

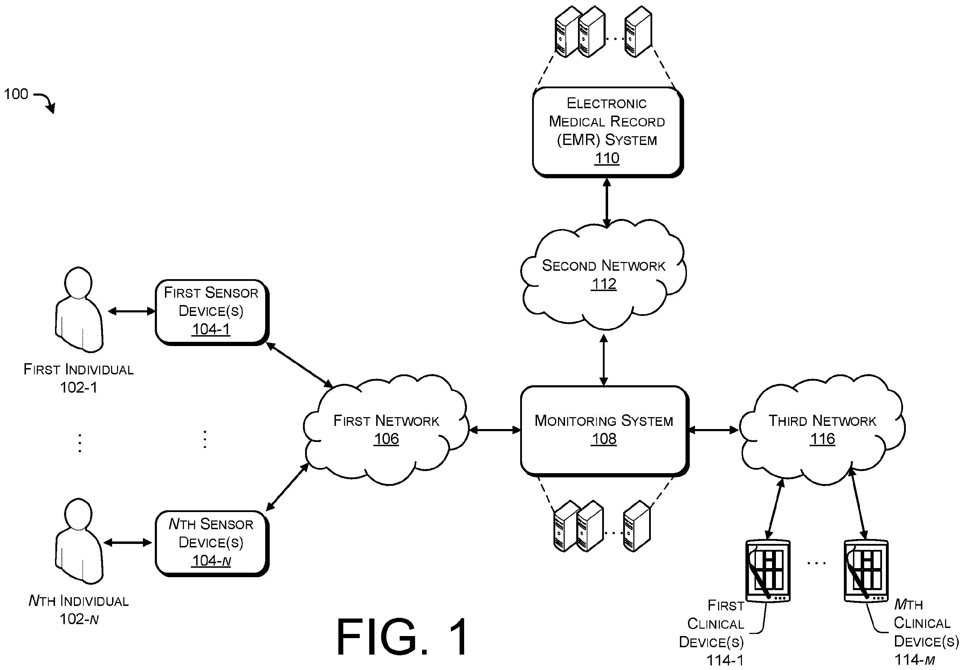

FIG. 1 illustrates an example environment 100 for continuously monitoring vital signs of multiple patients. The example environment 100 can be associated with a healthcare facility, in some cases. As shown with respect to at least FIG. 1, first through nth individuals 102-1 to 102-n can be monitored by first through nth sensor devices 104-1 to 104-n, wherein n is a positive integer. As used herein, the terms "individual," "patient," and their equivalents, can refer to a person who may be being actively monitored by a healthcare facility. In some implementations, the individuals 102-1 to 102-n can be individuals residing at least temporarily in a healthcare facility. For instance, the first individual 102-1 may be an individual who may be being monitored after a surgical procedure. In some implementations, the individuals 102-1 to 102-n may be monitored inside of an ICU, outside of an ICU, or in some other environment providing constant care and oversight from healthcare providers. For instance, the individuals 102-1 to 102-n may be monitored in one or more medical wards.

The sensor devices 104-1 to 104-n may include sensors that measure, sense, detect, and/or otherwise determine various conditions of the individuals 102-1 to 102-n. As used herein, the terms "sensor," "biological sensor," "vital sign sensor," and their equivalents, can refer to a device configured to measure, sense, detect, and/or otherwise determine a biological condition of an individual. As used herein, the terms "biological condition," "condition," and their equivalents, can refer to a physical parameter of an individual's body or a metric based on a physical measurement of the individual's body. In some examples a biological condition can be a vital sign (e.g., blood pressure, heart rate, temperature, respiration rate, blood oxygen saturation, etc.).

In some cases, sensors can further report the measured biological conditions to one or more external devices. Some examples of sensors include pressure sensors (e.g., pressure sensors installed in a hospital bed to sense the position of an individual resting in the hospital bed), blood-pressure sensors (e.g., a blood pressure sensor including a blood pressure cuff, a sensor measuring blood pressure based on pulse wave velocity, or the like), heart rate sensors (e.g., a smartwatch sensor), body temperature sensors (e.g., an infrared thermometer), respiratory rate sensors (e.g., a respiratory rate monitor), end-tidal CO₂ sensors, blood oxygen saturation sensors (e.g., pulse oximeters and/or other sensors that measure peripheral oxygen saturation (SpO₂) in arterial blood), chemical sensors (e.g., glucose sensors), electrical sensors (e.g., Electrocardiogram (ECG) sensors, Electroencephalogram (EEG) sensors, etc.), consciousness sensors (e.g., devices prompting individuals to engage in interactive mental exercises), or the like. Some sensors in the sensor devices 104-1 to 104-n may include movement sensors (e.g., gyroscopes, accelerometers, etc.), position sensors (e.g., pressure sensors on hospital beds of the individuals 102-1 to 102-n), or the like.

According to some implementations, the sensor devices 104-1 to 104-n can measure the conditions multiple times in a time interval. In some cases, the sensor devices 104-1 to 104-n can sample the conditions semi-continuously or periodically. As used herein, the term "semi-continuously" can refer to measurements performed at a sampling rate that meets or exceeds a minimum sampling rate defined according to the Nyquist theorem. In some examples, any one of the sensor devices 104-1 to 104-*n* may have a sampling rate of approximately 1-100 samples per minute, approximately 1-100 samples per hour, approximately 1-100 samples per day, or the like.

The sensor devices 104-1 to 104-*n* can transmit data indicating the measurements over a first network 106 to a monitoring system 108. In some cases, the sensor devices 104-1 to 104-*n* can measure various parameters (e.g., pressure, temperature, pulse frequency, electrical signals, etc.) associated with the individuals 102-1 to 102-*n*, generate signals indicating the measurements based on the parameters, and transmit the signals to the monitoring system 108. In some cases, the measurements can be derived by the sensor devices 104-1 to 104-*n* and/or the monitoring system 108 based on the parameters. As used herein, the term "network," and its equivalents can refer to any suitable communication network by which multiple nodes can exchange data. Examples of networks include Wide Area Networks (WANs), such as the Internet; Personal Area Networks (PANs); Local-Area Networks (LANs), Metropolitan-Area Networks (MANs), and the like. Networks can be wireless, such that nodes can communicate with each other over one or more wireless protocols, such as WLAN, Wi-Fi, Bluetooth, and the like. In some cases, nodes can exchange data in the form of one or more data packets. For example, the sensor devices 104-1 to 104-n can generate data packets by packaging the data indicating the measurements into data packets and transmit the data packets to the monitoring system 108 over the first network 106.

The monitoring system 108 may be configured to analyze the measurements and selectively report indications of the measurements. According to various implementations, the monitoring system 108 may time-average measurements and report the averages. In other words, the monitoring system 108 may report an average of the previous a measurements taken during a time interval, wherein a is a positive integer. For instance, the monitoring system 108 may report the arithmetic mean of measurements taken within the previous hour. In some examples, the monitoring system 108 may refrain from reporting every one of the measurements. For instance, the monitoring system 108 may identify multiple measurements of the first individual's 102-1 blood pressure determined over the course of a time period (e.g., one hour), determine an average of the first individual's 102-1 blood pressure over the time period, and report the average without reporting each of the measurements.

The monitoring system 108 may determine personalized baselines associated with the individuals 102-1 to 102-*n* based on measurements taken by the sensor devices 104-1 to 104-*n*. For instance, a baseline associated with the first individual 102-1 may be determined based on measurements taken by the first sensor device(s) 104-1. In some cases, each of the baselines can be identified based on multiple measurements taken during a time period, such as one hour to six hours, or the like. For example, the baseline associated with the first individual 102-1 may be an average or a median of multiple measurements taken by the first sensor device(s) 104-1 during the time period. In some cases, the baseline may be defined as the average if the measurements have a symmetric distribution and may be defined as the median if the measurements have an asymmetric distribution. As used herein, the terms "symmetric distribution," "Gaussian distribution," "normal distribution," and the like, can refer to a set of samples with a sample skewness whose absolute value is less than a predetermined threshold, such as 0.25. As used herein, the terms "asymmetric distribution," "skewed distribution," and the like, can refer to a set of samples with a sample skewness whose absolute value is greater than or equal to a predetermined threshold, such as 0.25.

In some examples, the monitoring system 108 may confirm that the multiple measurements during the time period are stable. For instance, the monitoring system 108 may identify that a variance of the multiple measurements may be below a particular level (e.g., 5-10% of the average of the multiple measurements, or the like). The monitoring system 108 may refrain from calculating the baseline if the measurements are unstable (e.g., if the variance is above the particular level).

In some instances, the monitoring system 108 may confirm that the individual is not moving, or is only moving a minimal amount, during the time period. For instance, an individual may be moving "a minimal amount" when the individual is determined to be moving less than a threshold distance (e.g., less than six inches), less than a threshold velocity or speed (e.g., less than one meter per second), less than a threshold acceleration (e.g., less than one meter per second squared), or the like. The sensor devices 104-1 to 104-n may include one or more movement sensors attached to the individuals 102-1 to 102-*n.* For instance, the sensor devices 104-1 to 104-*n* may include at least one accelerometer and/or at least one pressure sensor in at least one bed of the individuals 102-1 to 102-*n*. The movement sensors may measure movement of the individuals 102-1 to 102-*n* and report the measurements of the movement to the monitoring system 108 over the first network 106. Because movement can impact the measurement of various biological conditions, the monitoring system 108 may refrain from setting baselines for a biological condition measured during time frames in which the individuals 102-1 to 102-*n* are moving a non-minimal amount. The monitoring system 108 may compare the measurements of the movement to a threshold (e.g., a threshold distance, a threshold speed, a threshold velocity, a threshold acceleration, or the like), and only identify the baseline based on measurements during a time period in which the measurements of the movement are below the threshold. In various implementations, a movement threshold associated with a biological condition may be determined by monitoring movement and biological condition measurements of at least one individual. The movement threshold may be a maximum movement measurement associated with an accurate biological condition measurement. Accordingly, the movement threshold may be a maximum amount of movement that does not reduce the accuracy of the biological condition measurements.

In some examples, the monitoring system 108 may confirm that the individual maintains a position during the time period. The sensor devices 104-1 to 104-*n* may include one or more position sensors associated with the individuals 102-1 to 102-*n*. For example, the sensor devices 104-1 to 104-*n* may include at least one gyroscope at least one pressure sensor, and/or at least one accelerometer in at least one bed of the individuals 102-1 to 102-*n*, wherein measurements from the gyroscope(s), pressure sensor(s), and/or accelerometer(s) can be used to identify the posture and/or position of the individuals 102-1 to 102-*n*. The position sensors may measure the positions of the individuals 102-1 to 102-*n* in real time and report the position measurements to the monitoring system 108. The monitoring system 108 may selectively establish baselines based on biological condition measurements taken when the individuals 102-1 to 102-*n* are in stable positions. For instance, the monitoring system 108 may confirm, using the position measurements, that the first individual 102-1 is not changing position during a particular time period and may identify a baseline biological condition based on measurements taken during the particular time period. In some cases, the monitoring system 108 may selectively establish baselines based on biological condition measurements taken when the individuals 102-1 to 102-*n* are in a predetermined position (e.g., a position in the individuals 102-1 to 102-*n* will be in during later monitoring). For example, the monitoring system 108 may confirm, using the position measurements, that the first individual 102-1 is in a supine position during a certain time period and may identify a baseline biological condition based on measurements of the first individual 102-1 taken during the certain time period.

In some instances, the monitoring system 108 may confirm that the individuals 102-1 to 102-*n* have not been administered with a medication (e.g., a drug, medicine, biologic, pharmaceutical, or the like) that actively changes the individual's biological condition during the time period. For example, when albuterol is administered to an individual, the individual's heart rate will increase. In some examples, the monitoring system 108 may confirm that the individual has not been administered with the medicine during the time period. If there is a delay between administration and when the medication affects the biological condition, the monitoring system 108 may confirm that the medication's effect on the biological condition does not occur during the time period. For instance, some forms of albuterol have an onset within 15 minutes of administration, a peak effect 60-90 minutes after administration, and may be active for up to 5 hours after administration.

In some example implementations, the monitoring system 108 may determine that the first individual 102-1 has been administered with a particular medication (e.g., active ingredient, amount, dosage form, and the like) and a time at which the first individual 102-1 has been administered with the particular medication (i.e., an administration time). The monitoring system 108 may identify the medication and the administration time in any number of ways. For instance, the monitoring system 108 may be in communication with an Electronic Medical Record (EMR) system 110 over a second network 112. The EMR system 110 may store various health record information of the individuals 102-1 to 102-*n* and selectively transmit data indicating the health record information to the monitoring system 108. In various examples, the monitoring system 108 may access the EMR system 110 to identify the medication and/or the administration time of the first individual 102-1.

The monitoring system 108 may predict when the medication will be active for the first individual 102-1. For example, the monitoring system 108 may predict a time interval in which the medication is bioavailable to the first individual 102-1. In some cases, the monitoring system 108 may access a local database that indicates time intervals between administration and onset and/or durations of various medications including the medication administered to the first individual 102-1. Based on the administration time of the medication, the time interval between administration and onset of the medication, and/or the duration of the medication, the monitoring system 108 may confirm that the medication administered to the first individual 102-1 is not active during the time interval in which the measurements of the biological condition are taken. Accordingly, the monitoring system 108 may identify the baseline of the biological condition for the first individual 102-1 based on the measurements. In some cases, the monitoring system 108 may refrain from setting a baseline of a biological condition based on measurements of the biological condition taken during a time period when a medication is likely to be active and/or affect the biological condition.

As used herein, the term "confounding factor" can refer to at least one of a medical treatment, a surgical treatment, an administered medication, a bioactive medication, a movement by the individual, the individual's position, or the like, that can impact measurements of an individual's biological condition. In some examples, the monitoring system 108 may confirm that no confounding factor associated with the first individual 102-1 is present during a particular time period, and may identify a baseline of a biological condition for the first individual 102-1 based on measurements of the biological condition during the particular time period.

In some implementations, the monitoring system 108 may selectively set the baselines of the individuals 102-1 to 102-*n* based on measurements taken when the individuals 102-1 to 102-*n* are in states consistent with future monitoring. For instance, if the first individual 102-1 is expected to be treated with an opiate during future monitoring, the monitoring system 108 may identify the baseline respiratory rate of the first individual 102-1 based on respiratory rate measurements taken when the first individual 102-1 is administered with the opiate. In some cases, the monitoring system 108 may refrain from setting a baseline respiratory rate of the first individual 102-1 when the first individual 102-1 is not administered with the opiate. Accordingly, the monitoring system 108 may identify the baselines according to conditions that the individuals 102-1 to 102-*n* will be in during monitoring.

In various examples, the monitoring system 108 may determine the baselines using previous measurements of the biological conditions of the individuals 102-1 to 102-*n*. For example, the monitoring system 108 may further analyze the measurements made by the sensor devices 104-1 to 104-*n* in view of the health record information. For instance, the monitoring system may access the EMR system 110 to identify a measurement of the biological condition 108 during an intake procedure of the individual, measurements of the biological condition during a previous hospitalization of the individual, a measurement of the biological condition during a previous primary care visit of the individual, or the like.

In various implementations, the monitoring system 108 may further identify a range of the biological condition based on the baseline. In various implementations, a "primary range," a "baseline range," a "personalized range," or the like, for an individual may be a range of measurements of a biological condition that may be at least partially bounded by a baseline-dependent threshold. The range may be bounded by an upper threshold and a lower threshold. In various implementations, the monitoring system 108 may identify at least one of the upper threshold or the lower threshold based on the baseline.

In some cases, at least one of the thresholds may be determined by subtracting or adding an offset to the baseline. The offset may be a predetermined percentage of the baseline. For instance, the offset can be 20%, 10%, 5%, or some other percentage of the baseline. In some cases, the offset may be a fixed amount. In some examples, the offset may be 40, 30, 20, 10, 5, or some other number of the units of the baseline metric. For instance, if the baseline is a baseline blood pressure value in mmHg or kPa, the offset may be 30, 20, 10, 5, or some other value in mmHg or kPa. In some cases, the offset may be identified based on a maximum or minimum value of the biological condition and the baseline. For example, the offset may be equal to a fixed percentage of the absolute value of the difference between the minimum or maximum and the baseline. In some cases, the fixed percentage can be 10%, 20%, 30%, 40%, 50%, 60%, 70%, or some other percentage. For instance, if a baseline heart rate is 40 beats per minute, and a minimum heart rate is 0 beats per minute, the offset may be 25% of the difference between 40 beats per minute and 0 beats per minute, which is 10 beats per minute.

In some cases, the range may be identified based on a confidence interval associated with the measurements. For instance, the range may be defined as a 90% confidence interval, a 95% confidence interval, a 99% confidence interval, or the like, calculated from stable biological condition measurements.

In various examples, the monitoring system 108 may define at least one of the upper boundary or the lower boundary independently of the baseline. In some cases, a biological condition may be associated with a boundary that indicates that the individual may be unstable regardless of the individual's baseline. For instance, a heart rate of 40 beats per minute may be a minimum lower boundary, and a heart rate of 200 beats per minute may be a maximum upper boundary, regardless of the individual's baseline heart rate. Thus, the monitoring system 108, in some cases, can define a boundary of the range based on predetermined levels that are associated with unhealthy biological conditions for any individual in the population.

The monitoring system 108 may monitor additional measurements of the biological condition from the sensor devices 104 associated with the individual 102 in view of the baseline and/or the static or dynamic range of the biological condition. In some cases, the monitoring system 108 may trigger an alarm in response to identifying that at least one measurement of the biological condition of the individual may be outside of the range. For instance, the monitoring system 108 may average measurements of the biological condition over a particular time (e.g., one hour), and may trigger the alarm in response to identifying that the average may be outside of the range. In some cases, the monitoring system 108 may trigger the alarm in response to identifying that a predetermined number (e.g., 5) of measurements of the biological condition are outside of the range in a particular time period (e.g., one hour).

In some examples, the monitoring system 108 may identify a dynamic range for monitoring measurements of a biological condition of at least one of the individuals 102-1 to 102-n. In various instances, a predetermined trend may exist between a medical characteristic of the individual 102 and the biological condition. For example, albuterol may increase an individual's heart rate after administration. In some examples, a pain reliever (e.g., an opiate) may reduce an individual's blood pressure after administration. In some cases, a surgery may increase an individual's heart rate for an extended period of time. As used herein, a "medical characteristic" of an individual may refer to at least one of a medical treatment performed on the individual, a surgical treatment performed on the individual, a medicine administered to the individual, a dosage of the medicine administered to the individual, a biological condition (e.g., a vital sign) of the individual, an allergy of the individual (e.g., an allergy to a particular medicine), a genetic predisposition of the individual (e.g., a genotype and/or transcriptome of the individual), or the like.

In various implementations of the present disclosure, the monitoring system 108 may identify a trend between one or more medical characteristics and a biological condition. In some examples, the monitoring system 108 may receive, from the EMR system 110, a variety of health record information of a variety of previous patients. The monitoring system 108 may train a neural network to identify trends between medical characteristics and biological conditions using the health record information of the previous patients. After the neural network is trained, the monitoring system 108 may identify a mathematical relationship between one or more particular medical characteristics and a particular biological condition using the trained neural network. In some instances, the mathematical relationship may be predetermined and stored in the monitoring system 108. For example, a mathematical relationship between a biological condition and the administration of a particular medicine may be identified based on a predetermined pharmacokinetic model of bioavailability of the particular medicine.

The monitoring system 108 may receive an indication of one or more medical characteristics of an individual 102. In some cases in which the one or more medical characteristics include at least one other biological condition, a measurement of the other biological condition may be received from the sensor devices 104. In some examples, indications of the one or more medical characteristics can be received from the EMR system 110. For example, the monitoring system 108 may access the EMR system 110 determine that a particular medicine was administered at a particular time.

Using the mathematical relationship between the one or more medical characteristics and the biological condition, the monitoring system 108 may adjust the primary range (e.g., adjust the upper threshold and/or the lower threshold) of that biological condition based on the indications of the one or more medical characteristics. For instance, a mathematical relationship between albuterol dosage and time after administration can be used to increase the lower threshold and/or the upper threshold of a heart rate range for the individual 102 within an hour after the individual was administered with albuterol.

In some instances, the monitoring system 108 may widen or narrow the range, or adjust an offset of the range, based on one or more medical characteristics. A medical characteristic may be associated with increased risks for a particular pathologic outcome. For instance, an individual administered with an opiate may be at an increased risk for breathing irregularities. In an example, an individual with Marfan syndrome may be at an increased risk for heart damage resulting from high blood pressure. According to various implementations, the monitoring system 108 may store various rules linking medical characteristics to offsets and may adjust primary ranges based on those rules. For example, the monitoring system 108 may automatically increase the lower threshold of the individual's 102 respiration rate range in response to identifying that an opiate has been administered to the individual 102 within the past four hours.

In various implementations, the monitoring system 108 may further monitor a rate-of-change of the additional measurements. For example, the monitoring system 108 may calculate a running average of the rate-of-change of the additional measurements over a given time period (e.g., an hour). The monitoring system 108 may identify a range of the rate-of-change that represents normal conditions. For example, the range of the rate-of-change may be identified as a change of greater than ±1 %, ±5%, ±10%, ±20%, ±30%, or some other percentage range of the baseline over the course of a minute, an hour, or the like. In some instances, the range of the rate-of-change may be defined as a 90%, 95%, 99%, or some other percentage confidence interval of previously identified rates-of-change associated with a previous sent of measurements of the individual.

If the monitoring system 108 identifies that at least one rate-of-change of the measurements of the biological condition may be outside of the range, the monitoring system 108 may trigger the alarm. For instance, the monitoring system 108 may trigger the alarm in response to identifying that the average rate-of-change is outside of the range. As used herein, an alarm may be "triggered" when the monitoring system 108 causes output of a signal associated with the alarm. For instance, the monitoring system 108 may output an indication of the alarm on a monitor (or some other output device associated with the monitoring system 108), the monitoring system 108 may generate and transmit a signal indicating the alarm to an external device that outputs the indication of the alarm, or the like. In some cases, the monitoring system 108 may trigger the alarm in response to identifying that a predetermined number (e.g., five) of measurements of the biological condition maintain a rate-of-change that are outside of the range in a particular time period (e.g., one hour).

In some cases, the monitoring system 108 can trigger the alarm when one of the biological condition measurements or the rate-of-change is outside of its respective range and/or when both the biological condition measurements and the rate-of-change are outside of their respective ranges. For instance, the monitoring system 108 may selectively trigger the alarm when the biological condition is measured outside of a first range and/or the rate-of-change is outside of a second range. In some cases, the monitoring system 108 may trigger the alarm when one of the biological condition is outside of the first range and the rate-of-change is outside of the second range. In some cases, the monitoring system 108 selectively triggers the alarm when multiple biological conditions (and/or associated rates-of-change) are outside of their respective ranges.

In some examples, the monitoring system 108 may refrain from triggering the alarm if the monitoring system 108 identifies the presence of one or more confounding factors during a time period within which the biological condition measurements are measured. Accordingly, the monitoring system 108 may refrain from triggering the alarm when abnormal biological condition measurements and/or rates-of-changes may be caused by at least one confounding factor.

The monitoring system 108 may be in communication with first to *m*th clinical devices 114-1 to 114-*m* over a third network 116, wherein *m* is a positive integer. In various implementations, the monitoring system 108 may selectively report information about the individuals 102-1 to 102-*n* to the clinical devices 114-1 to 114-*m.* The information may be indicated in data transmitted from the monitoring system 108 to the clinical devices 114-1 to 114-*m*. In some cases, the monitoring system 108 may control, or otherwise cause at least one user interface associated with the clinical devices 114-1 to 114-*m*.

The monitoring system 108 may trigger the alarm by transmitting message(s) (e.g., signal(s)) to the first to *m*th clinical devices 114-1 to 114-*m*. In some cases, the message(s) may indicate the individual (e.g., the first individual 102-1) whose biological condition may be outside of the range. For instance, the message(s) may indicate an identity (e.g., a name, patient identifier, or the like) of the individual or a location (e.g., a bed identifier, a room identifier, or the like) of the individual. In some examples, the message(s) may indicate the biological condition whose measurement and/or rate-of-change may be outside of an applicable range.

According to some examples, the monitoring system 108 may cause at least one of the clinical devices 114-1 to 114-m to output a warning or an alert in response to determining that at least one biological condition of the individual is variant (e.g., at least one biological condition measurement and/or rate-of-change is outside of a primary range, is below a lower threshold, is above an upper threshold, or the like). As used herein, the term "warning" may refer to a signal that can be output from an electronic device indicating that an individual may be deteriorating, but the individual may not be in need of immediate (e.g., bedside) assistance. As used herein, the term "alert" may refer to a signal that can be output from an electronic device indicating that an individual may be in medical distress and in need of immediate assistance. In some cases, the monitoring system 108 may only cause the clinical devices 114-1 to 114-m to output the warning or the alert in response to determining that multiple biological conditions are variant. For instance, the monitoring system 108 may instruct the first clinical device 114-1 to output an alert associated with the first individual 102-1, when both a heart rate and a blood pressure of the first individual 102-1 are determined to be outside of primary ranges for the first individual 102-1, but may refrain from instructing the first clinical device 114-1 to output the alert when the heart rate may be outside of a primary range for the first individual 102-1 but the blood pressure may be within a primary range for the first individual 102-1. In some examples, the monitoring system 108 may instruct the first clinical device 114-1 to output an alert associated with the first individual 102-1 when both a heart rate and a temperature of the first individual 102-1 are determined to be outside of the primary ranges for the first individual 102-1 (e.g., indicating that the first individual 102-1 is septic), but may refrain from instructing the first clinical device 114-1 to output the alert when only one of the heart rate or the temperature is outside of its respective primary range. In some cases, the monitoring system 108 may indicate other levels associated with various patient statuses to the clinical devices 114-1 to 114-m.

The clinical devices 114-1 to 114-m may output warnings and/or alerts in various manners. In some cases, the clinical devices 114-1 to 114-*m* may output the warnings and/or alerts as auditory signals that can be heard by at least one care provider. For example, the clinical devices 114-1 to 114-*m* could be pagers indicating that a care provider should call another care provider or should attend an individual directly by outputting an audible ring. In some implementations, the clinical devices 114-1 to 114-*m* may output a warning and/or an alert as a haptic signal that can be felt by at least one care provider. For instance, the clinical devices 114-1 to 114-*m* may vibrate. According to various implementations, the clinical devices may output a warning and/or an alert as a visual signal. In some examples, the clinical devices 114-1 to 114-*m* may display pop-ups, blinking icons, colors, and various user interface elements portraying the alert. The alerts and warnings may be provided on various user interfaces of the clinical devices 114-1 to 114-*m*.

The warnings and/or alerts output by the clinical devices 114-1 to 114-*m* may prompt care providers to monitor the individuals 102-1 to 102-*n* associated with the warnings and/or alerts. In some cases, the warnings and/or alerts can be disabled by at least one care provider via the user interfaces. For instance, an alert indicating that the first individual 102-1 requires additional attention can be disabled in response to a care provider acknowledging the alert and/or reaching the first individual's 102-1 bedside.

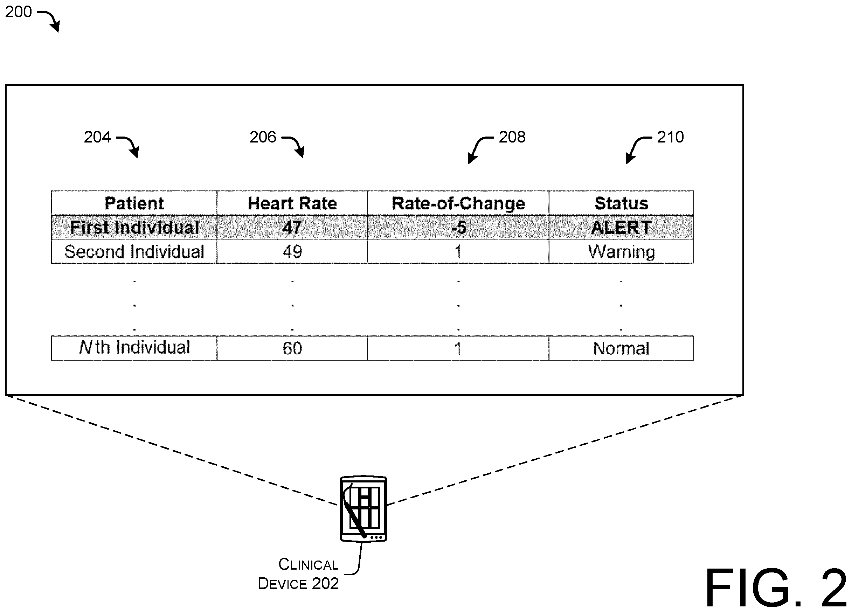

FIG. 2 illustrates an example of a visual interface 200 output by a clinical device 202. In some cases, the clinical device 202 illustrated in FIG. 2 can be any of the first to *m*th clinical devices 114-1 to 114-*m* described above with reference to FIG. 1.

In various implementations, the visual interface 200 may display statuses of various individuals being monitored in a clinical environment (e.g., a hospital). In some examples in which the clinical device 202 may be associated with (e.g., assigned to, carried by, or the like) a particular care provider, the visual interface 200 may display statuses of multiple (e.g., all) individuals whose care may be managed by the particular care provider. For instance, a nurse may be responsible for managing the care of 3-10 patients in a medical wards environment or 1-2 people in an ICU environment.

The visual interface 200 may display a table with multiple entries respectively corresponding to multiple individuals being monitored. Each entry may include four fields: a patient identifier 204, a vital sign 206, a rate-of-change 208, and a status 210. In a given entry, the patient identifier 204 may indicate a unique identifier of the individual being monitored. In some cases, the patient identifier 204 can include at least one of the individual's name, a location of the individual in the healthcare facility (e.g., a room identifier, a bed identifier, or the like), an identifier assigned to the individual upon intake (e.g., a unique string assigned to the individual), or the like.

In a given entry, the vital sign 206 may indicate a measurement and/or running average of measurements of a biological condition of an individual. In some cases, the vital sign 206 may be updated in real-time as measurements are being taken of the biological condition. In the implementation illustrated in FIG. 2, the vital sign 206 represents heart rate measurements (e.g., in beats per minute) of the individuals. In some examples, the vital sign 206 could additionally or alternatively represent a measurement and/or a running average of measurements of at least one of a blood pressure, a blood oxygen saturation percentage, a respiration rate, a core temperature, or a peripheral temperature of the individual. The rate-of-change 208 may represent a rate-of-change of the vital sign 206 over a given time interval. In some cases, the rate-of-change 208 may represent a rate-of-change between a current measurement (or average) displayed as the vital sign 206 and a previous measurement (or average) of the vital sign 206, divided by a time interval between times at which the current measurement (or average) and the previous measurement (or average) were taken (or a difference between time intervals over which the measurements were averaged). In various implementations, the rate-of-change 208 may be a running average of rates-of-change between measurements and/or averages. For instance, the rate-of-change 208 may correspond to an average rate-of-change of measurements taken over a previous hour, a previous two hours, a previous three hours, or the like. In the example illustrated in FIG. 2, the rate-of-change could be a rate-of-change in heart rate (e.g., in units of beats per square minute, beats per minute * hour, or the like).

The status 210 of a given entry may indicate whether an individual may be in need of assistance. Depending on the individual's vital sign 206 and/or rate-of-change 208, a monitoring system may identify whether the individual may be in need of assistance and cause the clinical device 202 to output the status 210 based on the identification. In the example illustrated in FIG. 2, the status 210 can have one of three levels: an alert level, a warning level, and a normal level. In various implementations, the status 210 can have one of any number of levels representing different levels of patient status, assistance required, severity of medical conditions experienced by individuals being monitored, or the like. When the status 210 is at the alert level, the individual may be in need of immediate or emergency assistance. When the status 210 is at the warning level, the individual may be deteriorating, but may not be in need of immediate assistance. When the status 210 is at the normal level, the individual may be stable and not in need of immediate assistance.

In the example illustrated in FIG. 2, the visual interface 200 may display statuses of first to nth individuals. In an entry corresponding to the first individual, the vital sign 206 may displayed as "47" (e.g., beats per minute) and the rate-of-change 208 may be displayed as "-5" (e.g., beats per square minute). In some instances, the first individual may have a lower threshold of 50 for the biological condition indicated by the vital sign 206 and may have a lower threshold of -4 for the rate-of-change 208. Because both the vital sign 206 and the rate-of-change 208 are below their respective lower thresholds, the first individual may be in need of immediate assistance. Accordingly, the status 210 may correspond to an "ALERT." In some cases, the color of the entry indicating the "ALERT" may be a different color than other entries corresponding to other statuses (e.g., "Warning" or "Normal").

In an entry corresponding to the second individual, the vital sign 206 may displayed as "49" (e.g., beats per minute) and the rate-of-change 208 may be displayed as "1" (e.g., beats per square minute). In some instances, the second individual may have a lower threshold of 50 for the biological condition indicated by the vital sign 206 and may have a lower threshold of -4 for the rate-of-change 208. Because the vital sign 206 of the second individual may be below its threshold, but the rate-of-change 208 may be above its threshold, the second individual may be deteriorating but not in need of immediate assistance. Accordingly, the status 210 may be displayed as a "Warning."

In an entry corresponding to the nth individual, the vital sign 206 may displayed as "60" (beats per minute) and the rate-of-change 208 may be displayed as "1" (beats per square minute). In some instances, the nth individual may have a lower threshold of 50 for the biological condition indicated by the vital sign 206 and may have a lower threshold of -4 for the rate-of-change 208. Because the vital sign 206 of the nth individual is below its threshold, but the rate-of-change 208 is above its threshold, the nth individual may not be deteriorating or in need of immediate assistance. Accordingly, the status 210 may be displayed as "Normal."

In various implementations, the visual interface 200 may display order of entries based on the statuses 210 of individuals corresponding to those entries. For instance, the monitoring system and/or the clinical device 202 may prioritize individuals based on statuses, and the visual interface 200 may display the order of the entries based on the priority of the corresponding individuals. In the example illustrated in FIG. 2, the first individual may have the highest priority because the first individual may be the only individual with an "ALERT" status. Accordingly, the entry corresponding to first individual may be displayed at the top of the list of entries in the visual interface 200. Further, the second individual may have a higher priority than the nth individual, because the second individual has a "Warning" status and the nth individual has a "Normal" status. Accordingly, the entry corresponding to the second individual may be displayed above the entry corresponding to the nth individual.

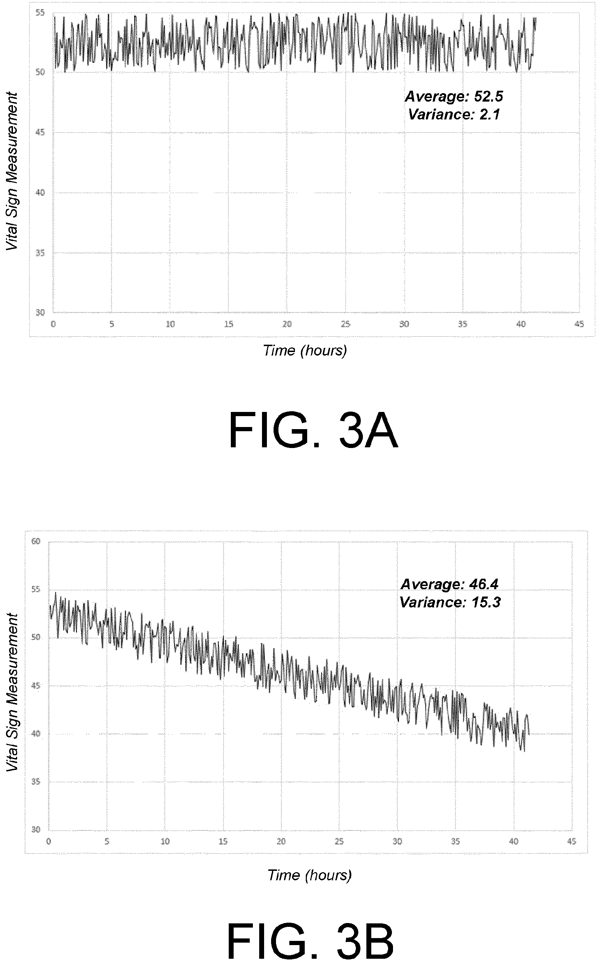

FIGS. 3A and 3B illustrate examples of vital sign measurements taken semi-continuously over periods of time. For instance, the vital sign measurements could represent heart rate in beats per minute. FIG. 3A illustrates an example of relatively stable vital sign measurements taken semi-continuously over a time period of about 42 hours. FIG. 3B illustrates an example of relatively unstable vital sign measurements taken semi-continuously over a time period of about 42 hours.

In various implementations, a monitoring system (e.g., the monitoring system 108) may receive and/or track the measurements of the vital sign over the time period. For instance, the monitoring system may receive the measurements from a sensor device (e.g., the sensor device 104) that measures the vital sign of an individual (e.g., the individual 102). The sensor device may have a sampling period that may be significantly shorter than the time period over which the measurements are taken. For instance, in the example illustrated in FIGS. 3A and 3B, the measurements are taken at a sampling period of 0.1 hours. The term "sampling period" may refer to a time period between measurements.

The measurements may vary over the course of the time period for various reasons. For instance, the sensor device may be associated with a level of sensor noise. The sensor noise may be attributable to a physical limitation of a sensor within the sensor device, a limitation of signal processing performed at the sensor device or the monitoring system, electronic noise introduced as signals representing the measurements travel through the sensor device, the monitoring system, or a communication interface between the sensor device and the monitoring system, or the like. In some cases, the measurements may vary over the time period due to the individual's deterioration and/or medical instability. Random variations in the vital sign measurements (e.g., due to noise) may insignificantly impact an average value of the measurements. However, variations due to a trend in the vital sign measurements (e.g., due to medical instability) may significantly impact an average value of the measurements.

The monitoring system may determine whether a baseline should be calculated based on the measurements. In some cases, the monitoring system may determine the baseline upon determining that the measurements are stable. For instance, the monitoring system may identify the variance of the measurements. If the variance of the measurements is less than a particular threshold (e.g., a particular percentage of the average of the measurements), the monitoring system may determine that the measurements are stable and may establish the baseline based on the measurements.

In some implementations, a monitoring system may identify any measurements with a variance of less than 10% of their average as stable and may determine a baseline based on those stable measurements. In the example illustrated in FIG. 3A, the measurements have an average of 52.5 and a population-based variance of 2.1. Accordingly, the monitoring system may identify a baseline based on the measurements depicted in FIG. 3A. In some cases, the baseline can be the average itself (i.e., 52.5). However, in the example illustrated in FIG. 3B, the measurements have an average of 46.4 and a variance of 15.3. Accordingly, the monitoring system may refrain from identifying a baseline based on the measurements depicted in FIG. 3B.

In some cases, despite the low variance of the measurements depicted in FIG. 3A, the monitoring system may nevertheless refrain from calculating the baseline from the measurements. For example, additional sensor data (not pictured) may indicate that the individual is moving during the time period in which the measurements are taken. In some instances, additional sensor data may indicate that the individual is in a position (e.g., a standing position) other than a predetermined position (e.g., a supine position), making the measurements inapplicable to the position in which the individual may be expected to be monitored (e.g., the supine position). In some examples, the monitoring system may determine (e.g., based on a medical record associated with the individual) that the individual has been dosed with a medication that affects the vital sign being measured. In some cases, if any of these features are determined to be applicable to the individual from which the measurements are taken, the monitoring system may refrain from calculating the baseline based on the measurements.

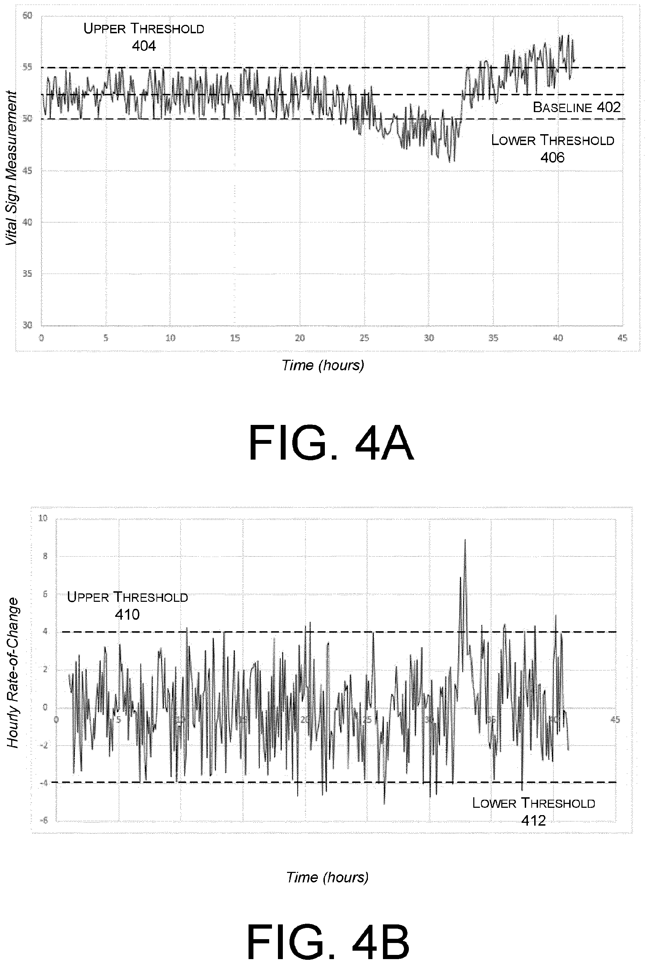

FIGS. 4A and 4B illustrate examples of vital sign measurements and rates-of-change of an individual. The vital sign measurements may be, for example, heart rate measurements in beats per minute. The rates-of-change may be defined in terms of hours. For instance, if the vital sign measurements are heart rate measurements, the rates-of-change may be identified as beats per minute hour (beats/(minute*hour)). In various implementations, a monitoring system can use the vital sign measurements and the rates-of-change to identify whether to trigger an alarm indicating that the individual may be in need of assistance. FIG. 4A shows vital sign measurements of the individual over a time period of about 42 hours. FIG. 4B shows a running average of the rates-of-change of the vital sign measurements illustrated in FIG. 4A, over the same about 42 hours depicted in FIG. 4A.

As illustrated in FIG. 4A, the vital sign measurements may be centered around a baseline 402 and vary within a range defined by an upper threshold 404 and a lower threshold 406, for about the first 23 hours depicted. However, the vital sign measurements may trend downward between 23 hours and 33 hours and may subsequently trend upward from 33 hours to the about 42 hours. During the downward trend, the vital sign measurements may dip below the lower threshold 406. During the upward trend, the vital sign measurements may rise above the upper threshold 404.

As illustrated in FIG. 4B, the hourly rates-of-change may generally remain between an upper threshold 410 and a lower threshold 412. However, at some time points the hourly rates-of-change may rise above the upper threshold 410 and dip below the lower threshold 412.

In various implementations, a monitoring system may determine whether to trigger an alarm based on the vital sign measurements and/or the hourly rates-of-change. In some cases, the monitoring system may trigger the alarm any time the vital sign measurements and/or the hourly rates-of-change fall outside their respective ranges. In some cases, the monitoring system may trigger the alarm any time the vital sign measurements dip below their lower threshold and the corresponding rates-of-change dip below their lower threshold, or any time the vital sign measurements rise above their upper threshold and the corresponding rates-of-change rise above their upper threshold. According to some implementations, if the vital sign measurements are low and trending downward, or high and trending upward, the alarm may be triggered.

Referring back to FIGS. 4A and 4B, in some cases, the vital sign measurements may dip below the lower threshold 406 at around the 24 hour mark, but the corresponding rates-of-change at the 24 hour mark are not below the lower threshold 412. Accordingly, in some cases, the monitoring system may refrain from triggering the alarm at the 24 hour mark. However, at around the 26 hour mark, the vital sign measurements dip below the lower threshold 405 and the corresponding rates-of-change dip below the lower threshold 412. Accordingly, in some examples, the monitoring system may trigger the alarm at the 26 hour mark.

In various examples, the monitoring system may identify that a confounding factor has occurred at the 26 hour mark, such as the individual may be moving, has changed position, or has been administered with a medication with a significant bioavailability at the 26 hour mark. The low vital sign measurements and downward trend at the 26 hour mark may be attributable to the event. Accordingly, in some of these examples, the monitoring system may refrain from triggering the alarm at the 26 hour mark.

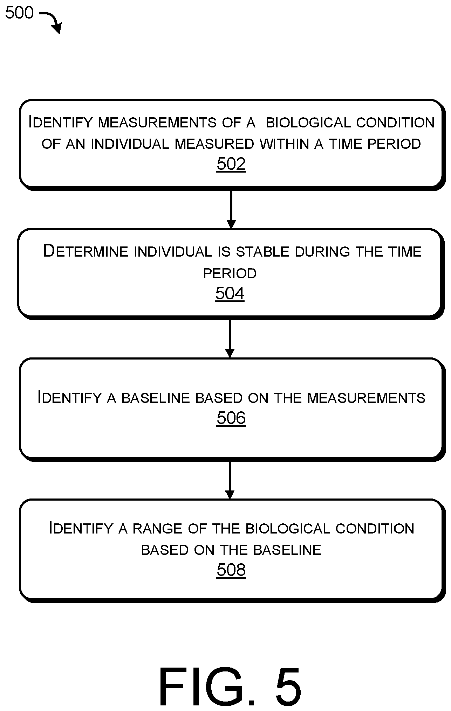

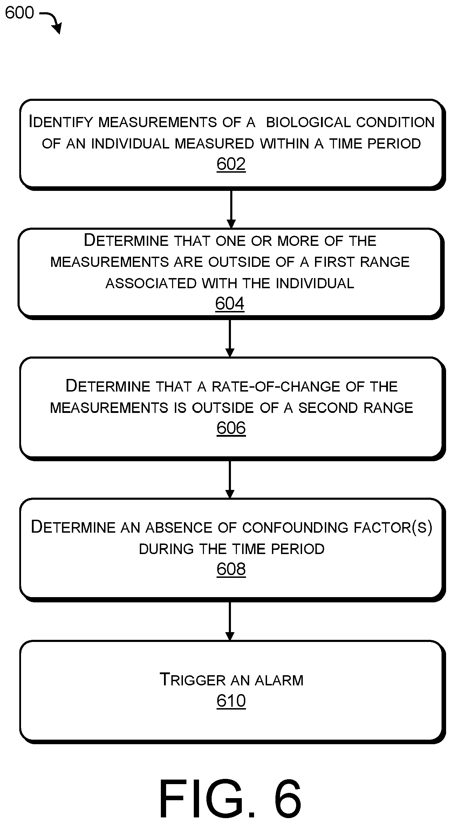

FIGS. 5 and 6 illustrate example processes in accordance with embodiments of the disclosure. These processes are illustrated as logical flow graphs, each operation of which represents a sequence of operations that can be implemented in hardware, software, or a combination thereof. In the context of software, the operations represent computer-executable instructions stored on one or more computer-readable storage media that, when executed by one or more processors, perform the recited operations. Generally, computer-executable instructions include routines, programs, objects, components, data structures, and the like that perform particular functions or implement particular abstract data types. The order in which the operations are described is not intended to be construed as a limitation, and any number of the described operations can be combined in any order and/or in parallel to implement the processes.

FIG. 5 illustrates an example process 500 for identifying a personalized range of biological condition measurements that can be used to monitor an individual. According to various implementations, the process 500 can be performed by a monitoring system, such as the monitoring system 108 described above with reference to FIG. 1. In some examples, at least some of the steps of the process 500 can be performed by at least one processor in a monitoring system.

At 502, the processor(s) may identify measurements of a biological condition of an individual that were measured within a time period. In various instances, the biological condition may be a vital sign. For example, the biological condition could be at least one of a heart rate, a blood pressure, a blood oxygen saturation percentage, a respiration rate, a core temperature, or a peripheral temperature of the individual.

In some instances, the processor(s) may receive the measurements from a sensor device that generates the measurements. For example, the processor(s) may be part of a system that has at least one transceiver configured to receive signals indicating the measurements from the sensor device, and the processor(s) may identify the measurements based on the signals. In some cases, the measurements are identified substantially in real-time. For instance, the sensor device may transmit the signals in response to generating the respective measurements.

According to various implementations, the processor(s) can identify multiple measurements of the biological condition that were taken during the time period. For example, the processor(s) can identify 5-100 measurements of the biological condition that were measured during the time period. A sampling period (e.g., a period of time between measurements) may be significantly shorter than a length of the time period over which the measurements were taken. For example, the length of the sampling period may be 5 to 100 times the length of the time period over which the measurements were taken. In some cases, the time period over which the measurements were taken can be within 0.5 to 24 hours. For instance, the time period can be within 1 to 6 hours.

At 504, the processor(s) may determine that the individual is stable during the time period. In various implementations, the processor(s) may determine that the measurements are stable during the time period. In some cases, the processor(s) may receive (e.g., via the transceiver(s)) a signal indicating that the individual may be stable from a separate system, such as an EMR system (e.g., the EMR system 110).

In various examples, the processor(s) may calculate a variance or a standard deviation of the measurements. The variance (or standard deviation) may be weighted or unweighted. In some cases, the processor(s) may compare the variance or standard deviation to a particular threshold. For example, the threshold may be 10 beats per minute if the variance is of heart rate measurements. If the variance or standard deviation is below the particular threshold, the processor(s) may determine that the individual may be stable.

In some implementations, the processor(s) may further calculate an average (e.g., an arithmetic mean) of the measurements and use the average to identify whether the individual may be stable. In various examples, the processor(s) may compare the variance or standard deviation to a particular percentage of the average. For example, the particular percentage may be 10% of the average. If the variance or standard deviation is below the particular percentage of the average, the processor(s) may determine that the individual may be stable.

Although not applicable to the example process 500 illustrated in FIG. 5, in some implementations, the processor(s) may identify that an individual may be unstable based on a signal from an external system and/or the measurements. In response to identifying that the individual may be unstable, the processor(s) may monitor additional measurements of the biological condition until the measurements are stable. In some examples, the processor(s) may generate a new set of measurements by omitting early measurements (e.g., the first one or more measurements in the time period over which the measurements have been taken) and repeat 504 based on the new set of measurements.

At 506, the processor(s) may identify a baseline based on the measurements. According to various implementations (e.g., when the measurements have a symmetric distribution), the baseline may be an average (e.g., an arithmetic mean) of the measurements. In some cases in which the measurements have a asymmetric distribution, the baseline may be a median of the measurements. For instance, measurements of a blood oxygen saturation percentage may have an asymmetric distribution, and a baseline blood oxygen saturation percentage may be a median of the measurements.

At 508, the processor(s) may identify a range of the biological condition based on the baseline. The range may be defined by a lower threshold and an upper threshold. The processor(s) may define at least one of the lower threshold or the upper threshold based on the baseline. In some cases, the processor(s) may identify the lower threshold or the upper threshold independently of the baseline. In various instances, the processor(s) may be configured to define the lower threshold or the upper threshold of a particular biological condition at a particular level. For example, the processor(s) may define the lower threshold of a range of heart rate measurements as 40 beats per minute, regardless of the individual's baseline.

In some cases, the processor(s) may calculate the threshold (e.g., the lower threshold or the upper threshold) by adding an offset value to the baseline. The offset value could be positive or negative. In some cases, the processor(s) applies the same offset value to every range of the same biological condition. For instance, the processor(s) may apply an offset value of 10 beats per minute to any range corresponding to a heart rate. In some instances, the processor(s) calculates the offset value based on the baseline itself. For example, the processor(s) may calculate a fixed percentage (e.g., 10%) of the baseline as an offset value. In various implementations, the processor(s) calculates both thresholds using two different offset values.

In some example implementations, the processor(s) may calculate a potential threshold based on the baseline but may not utilize the potential threshold if the potential threshold is too extreme. For instance, the processor(s) may calculate a potential lower threshold based on the baseline, compare the potential lower threshold to an absolute lower threshold, and use the potential lower threshold only if the potential lower threshold is greater than the absolute lower threshold. In some examples, the processor(s) may calculate a potential upper threshold based on the baseline, compare the potential upper threshold to an absolute upper threshold, and use the potential upper threshold only if the potential upper threshold is lower than the absolute upper threshold. The absolute thresholds may represent threshold biological condition measurements that should trigger an alarm for every individual, regardless of baseline. For example, an absolute lower threshold for heart rate measurements may be a minimum heart rate required to adequately oxygenate a human body and would apply to all individuals regardless of resting heart rate.

In some implementations, the processor(s) can compare additional measurements of the biological condition to the range in order to identify whether the individual's health may be deteriorating and/or whether the individual requires emergency assistance from a care provider.

FIG. 6 illustrates an example process 600 for triggering an alarm based on measurements of a biological condition. According to various implementations, the process 600 can be performed by a monitoring system, such as the monitoring system 108 described above with reference to FIG. 1. In some examples, at least some of the steps of the process 600 can be performed by at least one processor in a monitoring system.

At 602, the processor(s) can identify measurements of a biological condition of an individual that were measured within a time period. In various instances, the biological condition may be a vital sign. For example, the biological condition could be at least one of a heart rate, a blood pressure, a blood oxygen saturation percentage, a respiration rate, a core temperature, or a peripheral temperature of the individual.

In some instances, the processor(s) may receive the measurements from a sensor device that generates the measurements. For example, the processor(s) may be part of a system that has at least one transceiver configured to receive signals indicating the measurements from the sensor device, and the processor(s) may identify the measurements based on the signals. In some cases, the measurements are identified substantially in real-time. For instance, the sensor device may transmit the signals in response to generating the respective measurements.

According to various implementations, the processor(s) can identify multiple measurements of the biological condition that were taken during the time period. For example, the processor(s) can identify 5-100 measurements of the biological condition that were measured during the time period. A sampling period (e.g., a period of time between measurements) may be significantly shorter than a length of the time period over which the measurements were taken. For example, the length of the sampling period may be 5 to 100 times the length of the time period over which the measurements were taken. In some cases, the time period over which the measurements were taken can be within 0.01 to 24 hours. For instance, the time period can be within 0.1 to 1 hour.

At 604, the processor(s) can determine that one or more of the measurements are outside of a first range associated with the individual. The first range may have been previously defined (e.g., by the processor(s)) based on a baseline of previous measurements of the biological condition of the individual. For instance, the processor(s) may have previously identified the range by performing the process 500 described above with reference to FIG. 5.

In some cases, the processor(s) determines that at least a threshold number of measurements are outside of the first range. The measurements outside of the first range may be below the first range, above the first range, or a combination thereof. In example instances, the processor(s) may determine that 2 to 10 consecutive measurements are outside of the first range. In some instances, the processor(s) determines that the time period may be longer than a threshold time period (e.g., a threshold that may be about 0.1 to about 1 hour) and that all of the measurements taken within the time period are outside of the first range. In various examples, 604 includes the processor(s) determining that one or more averages (e.g., arithmetic means) of the measurements are outside of the first range.

At 606, the processor(s) can determine that a rate-of-change of the measurements may be outside of a second range. The rate-of-change outside of the second range may be below the first range or above the first range. The processor(s) may calculate the rate-of-change based on one or more of the measurements. In some cases, the processor(s) may calculate an average rate-of-change of at least a subset of the measurements. For instance, the processor(s) may calculate rates-of-change between each adjacent pair of the measurements and average the rates-of-change. In some instances, the processor(s) may calculate a rate-of-change based on a single pair of the measurements. For example, the processor(s) may calculate the rate-of-change based on the first pair of measurements that are outside of the first range, the last pair of measurements, or the like.

In some cases, the processor(s) may calculate the second range based on a confidence interval of previous rates-of-change calculated for the individual. For instance, the processor(s) may calculate multiple rates-of-change (e.g., running average rates-of-change, averaged over the course of an hour or some other time interval) based on previous measurements of the biological condition, and calculate a confidence interval based on the rates-of-change. The confidence level associated with the confidence interval may be 90%, 95%, 99%, or the like. The processor(s) may then define the second range based on the confidence interval. In some cases, the processor(s) may calculate the second range based on a predetermined level. For instance, the second range may be defined as a change of within ±5%, ±10%, ±15%, or the like, of the average of the measurements over a particular time interval (e.g., 1 hour). In some examples, the processor(s) may predetermine the second range based on the biological condition. For instance, the processor(s) may always define the second range to be ±10 beats per minute per 0.1 hour for heart rate measurements.

At 608, the processor(s) can determine the absence of any confounding factors present. A confounding factor can impact the individual's biological condition measurements, sometimes in predictable ways. Some examples of confounding factors include, for instance, recent medical treatments, surgical treatments, administered medicines, movement by the individual, changes of positions by the individual, or the like. In some cases, the processor(s) can access an EMR system (e.g., EMR system 110) to identify whether a confounding factor may be present during the time period, or within a predetermined prior time period before the time period in which the measurements have been taken. For example, the biological condition may be a heart rate, and the processor(s) may access the EMR system to determine whether the individual has been administered albuterol within a time range before the time period at which the heart rate measurements are taken.

Although not specifically illustrated in FIG. 6, in some implementations, the processor(s) can adjust the first range and/or the second range in response to identifying the presence of a confounding factor. In some cases, the processor(s) may identify a relationship between the biological condition and the identified factor and adjust the first range accordingly. For instance, the biological condition may be a heart rate, and the processor(s) may identify that the individual has been administered with albuterol and may raise the upper threshold and lower threshold of the first range in response to identifying that the individual has been administered with albuterol.

At 610, the processor(s) can trigger an alarm. In various implementations, the processor(s) may generate a message indicating an identity of the individual and/or a status of the individual, which may be transmitted to an electronic device via one or more transceivers. The electronic device may be associated with a care provider who can provide assistance to the individual. In some cases, the message may cause the electronic device to output an alert associated with the individual, such that the care provider may be informed that the individual requires immediate assistance.

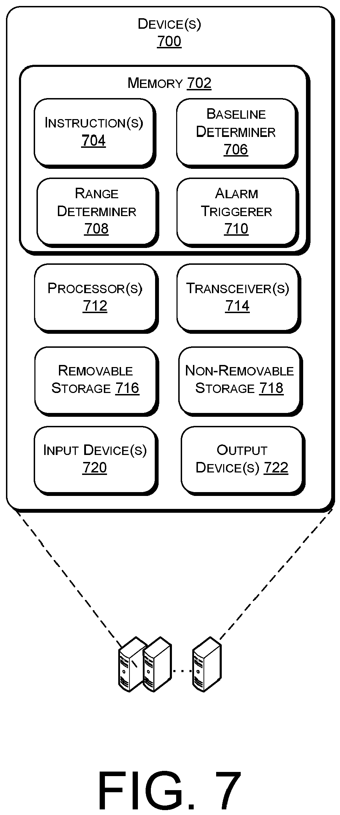

FIG. 7 illustrates an example system including at least one device 700. In some implementations, the system illustrated in FIG. 7 may perform any of the functionality described herein. The device(s) 700 may be implemented by at least one of server computer(s), dedicated hardware, software operating on dedicated hardware, or virtualized function(s) hosted on an appropriate platform (e.g., cloud infrastructure). The device(s) may be implemented as a single device or as multiple devices with components and data distributed among them.

As illustrated, the device(s) 700 comprise a memory 702. In various implementations, the memory 702 may be volatile (such as Random Access Memory (RAM)), non-volatile (such as Read Only Memory (ROM), flash memory, etc.) or some combination of the two. Various elements stored in the memory 702 can include methods, threads, processes, applications, objects, modules, or any other sort of executable instructions. Elements stored in the memory 702 may be non-transitory. The memory 702 may also store various files, databases, or the like.

The memory 702 may include various instructions 704, which may be for any of the functionality described herein. The memory 702 may also include a baseline determiner 706, a range determiner 708, and an alarm triggerer 710. The baseline determiner 706 may include instructions for determining personalized baselines based on measurements of various biological conditions (e.g., vital signs) of individuals. The range determiner 708 may include instructions for determining personalized ranges of biological conditions based on the personalized baselines. The alarm triggerer 710 may include instructions for monitoring measurements of biological conditions and triggering alarms when the measurements are outside of personalized ranges.

The instructions 704, baseline determiner 706, range determiner 708, and/or alarm triggerer 710 can be executed by processor(s) 712 to perform operations. In some embodiments, the processor(s) 712 includes a Central Processing Unit (CPU), a Graphics Processing Unit (GPU), or both CPU and GPU, or other processing unit or component known in the art.

As illustrated in FIG. 7, the device(s) 700 can also include one or more wired or wireless transceiver(s) 714. For example, the transceiver(s) 714 can include a Network Interface Card (NIC), a network adapter, a Local Area Network (LAN) adapter, or a physical, virtual, or logical address to connect to the various external devices and/or systems. The transceiver(s) 714 can include any sort of wireless transceivers capable of engaging in wireless communication (e.g., Radio Frequency (RF) communication). The transceiver(s) 714 can also include other wireless modems, such as a modem for engaging in Wi-Fi, WiMAX, Bluetooth, or infrared communication.

The device(s) 700 can also include additional data storage components such as, for example, magnetic disks, optical disks, or tape. These additional data storage components can include removable storage 716 and non-removable storage 718. Tangible computer-readable media can include volatile and nonvolatile, removable and non-removable media implemented in any method or technology for storage of information, such as computer readable instructions, data structures, program modules, or other data.

The memory 702, removable storage 716, and non-removable storage 718 are all examples of computer-readable storage media. Computer-readable storage media include, but are not limited to, RAM, ROM, EEPROM, flash memory or other memory technology, CD-ROM, Digital Versatile Discs (DVDs), Content-Addressable Memory (CAM), or other optical storage, magnetic cassettes, magnetic tape, magnetic disk storage or other magnetic storage devices, or any other medium which can be used to store the desired information and which can be accessed by the device(s) 700. Any such tangible computer-readable media can be part of the device(s) 700.

The device(s) 700 can also include input device(s) 720 and output device(s) 722. In some implementations, the input device(s) 720 can include at least one of a keypad, a cursor control, a touch-sensitive display, a voice input device, a haptic feedback device, or the like. The output device(s) 722 can include at least one of a display, speakers, a haptic output device, printers, etc. These devices are well known in the art and need not be discussed at length here.

In some instances, one or more components may be referred to herein as "configured to," "configurable to," "operable/operative to," "adapted/adaptable," "able to," "conformable/conformed to," etc. Those skilled in the art will recognize that such terms (e.g., "configured to") can generally encompass active-state components and/or inactive-state components and/or standby-state components, unless context requires otherwise.

As used herein, the term "based on" can be used synonymously with "based, at least in part, on" and "based at least partly on."

As used herein, the terms "comprises/comprising/comprised" and "includes/including/included," and their equivalents, can be used interchangeably. An apparatus, system, or method that "comprises A, B, and C" includes A, B, and C, but also can include other components (e.g., D) as well. That is, the apparatus, system, or method is not limited to components A, B, and C.

Embodiments of the invention can be described with reference to the following numbered clauses, with preferred features laid out in the dependent clauses:
1. A method, including: receiving, from a sensor, a first signal including information indicative of first measurements of a vital sign of an individual measured during a first time period; determining a baseline of the vital sign based on the first measurements; identifying a threshold of the vital sign based on the baseline; receiving, from the sensor, a second signal including information indicative of second measurements of the vital sign of the individual measured during a second time period; determining that at least one of the second measurements is outside of a range bounded by the threshold; and in response to determining that at least one of the second measurements is outside of the range, transmitting, to at least on clinical device, a third signal indicating an alarm.
2. The method of clause 1, wherein determining the baseline of the vital sign includes: identifying a variance of the first measurements; and determining that the variance is less than a predetermined percentage of an arithmetic mean of the first measurements, wherein the threshold is identified in response to determining that the variance is less than the predetermined percentage.
3. The method of clause 1 or 2, wherein the sensor is a first sensor and the threshold is a first threshold, the method further including: receiving, from a second sensor, a fourth signal including information indicative of third measurements of movement of the individual during the first time period; and determining that the third measurements are below a second threshold, wherein identifying the first threshold is in response to determining that the third measurements are below the second threshold.
4. The method of any of clauses 1 to 3, wherein the sensor is a first sensor and the threshold is a first threshold, the method further including: receiving, from a second sensor, a fourth signal including information indicative of third measurements of a position of the individual during the first time period; and determining that the individual has maintained a supine position during the first time period based on the third measurements, wherein identifying the first threshold is in response to determining that the individual has maintained the supine position.
5. The method of any of clauses 1 to 4, further including: determining that a medication administered to the individual is inactive during the first time period, wherein the threshold is identified in response to determining that the medication is inactive during the first time period.
6. The method of any of clauses 1 to 5, wherein the threshold is a first threshold and the range is a first range, the method further including: determining that a rate-of-change of the second measurements is outside of a second range that is bounded by a second threshold, wherein triggering the alarm is further in response to determining that the rate-of-change of the second measurements is outside of the second range.
7. The method of clause 6, wherein: the at least one of the second measurements is determined to be below the first range and the rate-of-change is determined to be below the second range, or the at least one of the second measurements is determined to be above the first range and the rate-of-change is determined to be above the second range.
8. The method of any of clauses 1 to 7, wherein the vital sign includes at least one of a heart rate of the individual, a blood pressure of the individual, a blood oxygen saturation percentage of the individual, a respiration rate of the individual, a core temperature of the individual, or a peripheral temperature of the individual.
9. A system, including: at least one processor; and memory storing instructions that, when executed by the at least processor, cause the at least one processor to perform operations including: receiving, from a sensor, a first signal indicating first measurements of a vital sign of an individual taken during a first time period; determining a baseline of the vital sign based on the first measurements; identifying a threshold of the vital sign based on the baseline; receiving, from the sensor, a second signal indicating second measurements of the vital sign of the individual taken during a second time period; determining that at least one of the second measurements is outside of a range bounded by the threshold; and in response to determining that at least one of the second measurements is outside of the range, triggering an alarm.
10. The system of clause 9, wherein the sensor is a first sensor and the threshold is a first threshold, the operations further including: receiving, from a second sensor, a third signal indicating third measurements of movement of the individual taken during the second time period; and determining that the third measurements are below a second threshold, wherein determining that at least one of the second measurements is outside of the range bounded by the threshold is in response to determining that the third measurements are below the second threshold.
11. The system of clause 9 or 10, wherein the sensor is a first sensor and the threshold is a first threshold, the operations further including: receiving, from a second sensor, a third signal indicating third measurements of a position of the individual taken during the second time period; and determining that the individual has maintained a supine position during the second time period based on the third measurements, wherein determining that at least one of the second measurements is outside of the range bounded by the threshold is in response to determining that the individual has maintained the supine position during the second time period.
12. The system of any of clauses 9 to 11, wherein the operations further comprise: determining that a medication administered to the individual is inactive during the second time period, wherein determining that at least one of the second measurements is outside of the range bounded by the threshold is in response to determining that the medication is inactive during the second time period.
13. The system of any of clauses 9 to 12, wherein the threshold is a first threshold and the range is a first range, the operations further including: determining that a rate-of-change of the second measurements is outside of a second range that is bounded by a second threshold, wherein triggering the alarm is further in response to determining that the rate-of-change of the second measurements is outside of the second range.
14. The system of clause 13, wherein: the at least one of the second measurements is determined to be below the first range and the rate-of-change is determined to be below the second range, or the at least one of the second measurements is determined to be above the first range and the rate-of-change is determined to be above the second range.
15. The system of any of clauses 9 to 14, wherein the threshold is a first threshold and determining that at least one of the second measurements is outside of the range bounded by the threshold includes: determining that greater than a second threshold number of the second measurements are outside of the range bounded by the first threshold.
16. A system, including: a vital sign sensor configured to measure a vital sign of an individual; a movement sensor configured to measure movement of the individual; an electronic device configured to output an alert; at least one processor; and memory storing instructions that, when executed by the at least one processor, cause the at least one processor to perform operations including: receiving, from the vital sign sensor, a first signal indicating first measurements of the vital sign measured during a first time period; receiving, from the movement sensor, a second signal indicating second measurements of the movement of the individual measured during the first time period; determining that the second measurements are below a first threshold; determining a baseline of the vital sign based on the first measurements by calculating an arithmetic mean of the first measurements; identifying a second threshold of the vital sign based on the baseline, the second threshold being a predetermined percentage of the baseline; receiving, from the vital sign sensor, a third signal indicating third measurements of the vital sign measured during a second time period; receiving, from the movement sensor, a fourth signal indicating fourth measurements of the movement measured during the second time period; determining that the fourth measurements are below the first threshold; determining that at least one of the third measurements is outside of a range bounded by the second threshold; and in response to determining that at least one of the third measurements is outside of the range, transmitting, to the electronic device, a fifth signal indicating an instruction to output an alarm identifying the individual.
17. The system of clause 16, wherein the range is a first range, and the operations further comprise: identifying an average rate-of-change of the third measurements; and determining that the average rate-of-change is outside of a second range, wherein the fifth signal is transmitted in response to determining that the average rate-of-change is outside of the second range.
18. The system of clause 17, wherein determining that the rate-of-change is outside of the second range includes determining that the average rate-of-change is greater than the second range, and wherein determining that at least one of the third measurements is outside of the first range includes determining that the at least one of the third measurements is greater than the second threshold.
19. The system of claim 17, wherein determining that the rate-of-change is outside of the second range includes determining that the average rate-of-change is less than the second range, and wherein determining that at least one of the third measurements is outside of the first range includes determining that the at least one of the third measurements is less than the second threshold.
20. The system of any of clauses 16 to 19, wherein the electronic device, in response to receiving the fifth signal, outputs the alarm as at least one of a visual alert, an auditory alert, or a haptic alert.

## Claims

1. A method carried out by a processor, comprising:
receiving, from a sensor, a first signal including information indicative of first measurements of a vital sign of an individual measured during a first time period;
determining a baseline of the vital sign based on the first measurements;
identifying a threshold of the vital sign based on the baseline;
receiving, from the sensor, a second signal including information indicative of second measurements of the vital sign of the individual measured during a second time period;
determining that at least one of the second measurements is outside of a range bounded by the threshold; and
in response to determining that at least one of the second measurements is outside of the range, transmitting, to at least on clinical device, a third signal indicating an alarm.

2. The method of claim 1, wherein determining the baseline of the vital sign comprises:
identifying a variance of the first measurements; and
determining that the variance is less than a predetermined percentage of an arithmetic mean of the first measurements,
wherein the threshold is identified in response to determining that the variance is less than the predetermined percentage.

3. The method of any preceding claim, wherein the sensor is a first sensor and the threshold is a first threshold, the method further comprising:
receiving, from a second sensor, a fourth signal including information indicative of third measurements of movement of the individual during the first time period; and
determining that the third measurements are below a second threshold,
wherein identifying the first threshold is in response to determining that the third measurements are below the second threshold.

4. The method of any preceding claim, wherein the sensor is a first sensor and the threshold is a first threshold, the method further comprising:
receiving, from a second sensor, a fourth signal including information indicative of third measurements of a position of the individual during the first time period; and
determining that the individual has maintained a supine position during the first time period based on the third measurements,
wherein identifying the first threshold is in response to determining that the individual has maintained the supine position.

5. The method of any preceding claim, further comprising:
determining that a medication administered to the individual is inactive during the first time period,
wherein the threshold is identified in response to determining that the medication is inactive during the first time period.

6. The method of any preceding claim, wherein the threshold is a first threshold and the range is a first range, the method further comprising:
determining that a rate-of-change of the second measurements is outside of a second range that is bounded by a second threshold,
wherein triggering the alarm is further in response to determining that the rate-of-change of the second measurements is outside of the second range.

7. The method of claim 6, wherein:
the at least one of the second measurements is determined to be below the first range and the rate-of-change is determined to be below the second range, or
the at least one of the second measurements is determined to be above the first range and the rate-of-change is determined to be above the second range.

8. The method of any preceding claim, wherein the vital sign comprises at least one of a heart rate of the individual, a blood pressure of the individual, a blood oxygen saturation percentage of the individual, a respiration rate of the individual, a core temperature of the individual, or a peripheral temperature of the individual.

9. A system, comprising:
at least one processor; and
memory storing instructions that, when executed by the at least processor, cause the at least one processor to perform operations comprising:
receiving, from a sensor, a first signal indicating first measurements of a vital sign of an individual taken during a first time period;
determining a baseline of the vital sign based on the first measurements;
identifying a threshold of the vital sign based on the baseline;
receiving, from the sensor, a second signal indicating second measurements of the vital sign of the individual taken during a second time period;
determining that at least one of the second measurements is outside of a range bounded by the threshold; and
in response to determining that at least one of the second measurements is outside of the range, triggering an alarm.

10. The system of claim 9, wherein the sensor is a first sensor and the threshold is a first threshold, the operations further comprising:
receiving, from a second sensor, a third signal indicating third measurements of movement of the individual taken during the second time period; and
determining that the third measurements are below a second threshold,
wherein determining that at least one of the second measurements is outside of the range bounded by the threshold is in response to determining that the third measurements are below the second threshold.

11. The system of either claim 9 or claim 10, wherein the sensor is a first sensor and the threshold is a first threshold, the operations further comprising:
receiving, from a second sensor, a third signal indicating third measurements of a position of the individual taken during the second time period; and
determining that the individual has maintained a supine position during the second time period based on the third measurements,
wherein determining that at least one of the second measurements is outside of the range bounded by the threshold is in response to determining that the individual has maintained the supine position during the second time period.

12. The system of any one of claims 9 to 11, wherein the operations further comprise:
determining that a medication administered to the individual is inactive during the second time period,
wherein determining that at least one of the second measurements is outside of the range bounded by the threshold is in response to determining that the medication is inactive during the second time period.

13. The system of any one of claims 9 to 12, wherein the threshold is a first threshold and the range is a first range, the operations further comprising:
determining that a rate-of-change of the second measurements is outside of a second range that is bounded by a second threshold,
wherein triggering the alarm is further in response to determining that the rate-of-change of the second measurements is outside of the second range.

14. The system of claim 13, wherein:
the at least one of the second measurements is determined to be below the first range and the rate-of-change is determined to be below the second range, or
the at least one of the second measurements is determined to be above the first range and the rate-of-change is determined to be above the second range.

15. The system of any of one claims 9 to 14, wherein the threshold is a first threshold and determining that at least one of the second measurements is outside of the range bounded by the threshold comprises:
determining that greater than a second threshold number of the second measurements are outside of the range bounded by the first threshold.
